# FASCICULE DE BREVET EUROPEEN

(11) **EP 1 765 432 B1**
(45) Date de publication et mention de la délivrance du brevet: **17.04.2013**
(21) Numéro de dépôt: 05763727.4
(22) Date de dépôt: 02.05.2005
(51) Int. Cl.: A61M 1/00

(54) **DISPOSITIF D'ASPIRATION POUR APPLICATIONS MÉDICALES**
SAUGVORRICHTUNG FÜR MEDIZINISCHE ANWENDUNGEN
SUCTION DEVICE FOR MEDICAL APPLICATIONS

(30) Priorité: 04.05.2004 FR 0404721
(43) Date de publication de la demande: 28.03.2007
(73) Titulaire: Boussignac, Georges, 92160 Antony (FR)
(72) Inventeur: Boussignac, Georges, 92160 Antony (FR)
(74) Mandataire: Coralis Harle
(86) Numéro de dépôt international: PCT/FR2005/001089
(87) Numéro de publication internationale: WO 2005/120599

(56) Documents cités:
- EP-A- 0 620 016
- GB-A- 234 623
- US-A- 3 499 393

## Description

La présente invention concerne un dispositif d'aspiration pour applications thérapeutiques qui, quoique non exclusivement, est particulièrement adapté au traitement des pneumothorax.

On sait que certaines thérapies nécessitent la mise en oeuvre de vide. Par exemple, le traitement d'un pneumothorax consiste à aspirer l'épanchement gazeux plaquant la plèvre contre le poumon.

Généralement, un tel vide est créé par une seringue d'aspiration, car il est rare que les hôpitaux -sauf éventuellement les salles d'opération- soient équipés en vide. De toute façon, les installations mobiles de soins d'urgence ne comportent aucune alimentation en vide.

En revanche, aussi bien les hôpitaux que les installations de soins mobiles d'urgence sont équipés de sources de gaz sous pression, notamment sous forme de bouteilles.

C'est ainsi qu'il a été proposé dans GB-234.623, US-3.499.393 ou EP-0.620.016 des dispositifs d'aspiration.

La présente a pour objet de mettre à profit cette dernière remarque pour mettre à disposition des praticiens des sources de vide simples, légères et peu onéreuses.

A cette fin, selon l'invention, le dispositif d'aspiration pour applications médicales comportant :
- un canal d'aspiration, dont l'extrémité distale est apte à être reliée à un patient ; et
- des moyens pour engendrer une dépression dans ledit canal d'aspiration à l'aide d'un gaz sous pression,
est remarquable en ce que :
- lesdits moyens pour engendrer ladite dépression comportent un orifice micrométrique disposé dans un canal de soufflage dont l'axe converge vers celui dudit canal d'aspiration, ledit orifice micrométrique étant alimenté en gaz sous pression du côté opposé audit canal d'aspiration ;
- le canal de soufflage converge vers l'extrémité proximale dudit canal d'aspiration, l'extrémité distale dudit canal de soufflage étant apte à être reliée à une source de gaz sous pression ;
- un canal d'évacuation est relié aux extrémités proximales dudit canal d'aspiration et dudit canal de soufflage pour prolonger ces deux canaux du côté proximal ; et
- l'extrémité proximale dudit canal d'évacuation est pourvue de moyens évitant son bouchage accidentel, lesdits moyens se présentant sous forme d'orifices latéraux percés à travers ledit canal d'évacuation.

Ainsi, le gaz sous pression sortant à grande vitesse dudit orifice micrométrique crée une dépression à l'extrémité proximale du canal d'aspiration, de sorte qu'il est possible d'aspirer des gaz et/ou des liquides d'un patient à travers ledit canal d'aspiration.

La dépression ainsi obtenue dépend de la pression et du débit du gaz sous pression, ainsi que du diamètre de l'orifice micrométrique. De façon générale, les bouteilles de gaz à usage médical délivrent leur gaz sous une pression de l'ordre de 3,5 bars (3,5 x 10⁵ pascals). Avec une telle pression -aisément disponible- et un diamètre de l'ordre de 300 microns pour l'orifice micrométrique, l'expérience montre que l'on obtient une dépression à l'extrémité proximale du canal d'aspiration de l'ordre de 80 millibars (80 x 10² pascals) avec un débit, c'est-à-dire une consommation, d'environ quatre litres de gaz par minute. Une telle dépression est satisfaisante pour les applications médicales, du type traitement de pneumothorax, et la consommation de gaz qui en résulte est compatible avec les bouteilles portatives de gaz sous pression, utilisées dans les installations de soins mobiles.

L'orifice micrométrique peut être formé dans un obturateur rapporté dans ledit canal de soufflage et/ou être formé par l'extrémité proximale d'un conduit micrométrique.

Dans ce dernier cas, c'est le conduit micrométrique qui est formé dans ledit obturateur.

Le dispositif peut comporter une pièce tubulaire en forme de Y, par exemple en matière plastique, dont les branches constituent respectivement le canal d'aspiration et le canal de soufflage et dont le tronc commun forme ledit canal d'évacuation.

Dans un mode de réalisation avantageux, le dispositif d'aspiration est associé à un récipient de collecte dans lequel pénètre l'extrémité proximale dudit canal d'évacuation et qui comporte au moins un évent. Un tel récipient de collecte peut présenter la forme d'une poche plastique et ledit dispositif d'aspiration peut en être solidaire. On obtient ainsi un ensemble dispositif d'aspiration - récipient de collecte à un seul usage, ne nécessitant pas d'être stérilisé après usage.
Les figures du dessin annexé feront bien comprendre comment l'invention peut être réalisée. Sur ces figures, des références identiques désignent des éléments semblables.
La figure 1 montre, en coupe schématique, un mode de réalisation du dispositif d'aspiration conforme à la présente invention.
La figure 2 est une coupe transversale du canal de soufflage selon la ligne II-II de la figure 1.
La figure 3 illustre, en fonctionnement, l'ensemble du dispositif d'aspiration de la figure 1 et d'une poche de collecte des fluides aspirés.

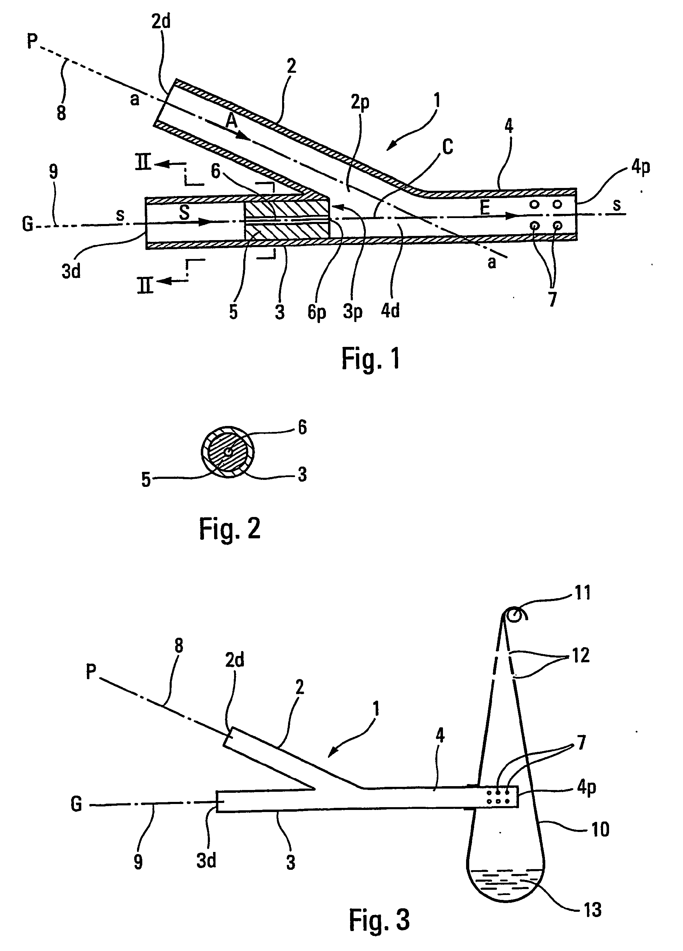

L'exemple de réalisation 1 du dispositif d'aspiration pour applications médicales, conforme à la présente invention et montré par les figures 1 et 2, comporte un canal d'aspiration 2, un canal de soufflage 3 et un canal d'évacuation 4. Les canaux 2, 3 et 4 forment une sorte de Y, dont les branches sont constituées respectivement par le canal d'aspiration 2 et le canal de soufflage 3 et dont le tronc commun est formé par le canal d'évacuation 4. A cet effet, les axes respectifs a-a et s-s du canal d'aspiration 2 et du canal de soufflage 3 convergent l'un vers l'autre et les extrémités proximales 2p et 3p des canaux 2 et 3, ainsi que l'extrémité distale 4d du canal d'évacuation 4, délimitent un espace commun C. L'axe du canal d'évacuation 4 peut être confondu avec l'axe s-s du canal de soufflage 3.

Les trois canaux 2, 3 et 4 peuvent former une unique pièce tubulaire à la forme d'un Y.

Dans le canal de soufflage 3 est disposé un obturateur 5, percé longitudinalement d'un conduit micrométrique 6, dont l'orifice proximal 6p débouche dans l'extrémité proximale 3p dudit canal de soufflage 3, c'est-à-dire qu'il est disposé au voisinage de l'extrémité proximale 2p du canal d'aspiration 2.

Au voisinage de son extrémité proximale 4p, le canal d'évacuation 4 est percé d'orifices latéraux 7, évitant son bouchage accidentel.

Par toute liaison connue 8, l'extrémité distale ouverte 2d du canal d'aspiration 2 peut être reliée à un patient P (non représenté). De même, par toute liaison connue 9, l'extrémité distale ouverte 3d du canal de soufflage 3 peut être reliée à une source de gaz sous pression G (non représentée), par exemple constituée par une bouteille de gaz comprimé.

Le débit de gaz sous pression pénétrant dans le canal de soufflage 3, comme cela est symbolisé par la flèche S, s'accélère fortement au passage à travers le conduit micrométrique 6, de sorte qu'une dépression dp est engendrée dans l'espace C commun aux extrémités proximales 2p et 3p et à l'extrémité distale 4d. Il en résulte une aspiration dans le canal d'aspiration 2, comme cela est illustré par la flèche A.

Par suite, les gaz et/ou les liquides devant être évacués du patient P sont aspirés dans le canal 2 et éliminés par le canal d'évacuation 4 (voir la flèche E).

Bien entendu, la valeur de la dépression dp, c'est-à-dire l'efficacité de l'aspiration A, dépend de la pression et du débit du gaz sous pression de la source G et de la longueur et du diamètre du conduit micrométrique 6.

L'expérience a montré qu'avec un débit de gaz de quelques litres par minute, sous une pression de quelques bars (quelques 10⁵ pascals), à la sortie de la source G, et un conduit 6 présentant une longueur de quelques centimètres et un diamètre de quelques centaines de microns, il est possible d'obtenir une valeur de quelques dizaines de millibars (quelques 10² pascals) pour la dépression dp.

Sur la figure 3, on a représenté le dispositif 1 avec l'extrémité proximale 4p du canal d'évaluation 4, y compris les trous latéraux 7, introduite dans une poche souple plastique 10, apte à être suspendue à une barre 11 et pourvue d'évents 12 à sa partie supérieure.

Le canal d'évacuation 4 et la poche plastique 10 peuvent être solidarisés l'un de l'autre, par exemple par soudure au niveau du passage dudit canal d'évacuation à travers la paroi de ladite poche.

On obtient ainsi un ensemble d'aspiration 1-10, apte à aspirer et recueillir (en 13) les gaz et/ou liquides d'un patient P en cours de traitement et pouvant être jeté après usage.

## Revendications

1. Dispositif d'aspiration (1) pour applications médicales, comportant :
- un canal d'aspiration (2), dont l'extrémité distale (2d) est apte à être reliée à un patient (P) ; et
- des moyens pour engendrer une dépression dans ledit canal d'aspiration (2) à l'aide d'un gaz sous pression,
**caractérisé en ce que** :
- lesdits moyens pour engendrer ladite dépression comportent un orifice micrométrique (6p) disposé dans un canal de soufflage (3) dont l'axe (s-s) converge vers celui (a-a) dudit canal d'aspiration (2), ledit orifice micrométrique (6p) étant alimenté en gaz sous pression du côté opposé audit canal d'aspiration (2) ;
- le canal de soufflage converge vers l'extrémité proximale (2p) dudit canal d'aspiration (2), l'extrémité distale (3d) dudit canal de soufflage (3) étant apte à être reliée à une source de gaz sous pression (G) ;
- un canal d'évacuation (4) est relié aux extrémités proximales (2p, 3p) dudit canal d'aspiration (2) et dudit canal de soufflage (3) pour prolonger ces deux canaux (2,3) du côté proximal ; et
- l'extrémité proximale (4p) dudit canal d'évacuation (4) est pourvue de moyens (7) évitant son bouchage accidentel, lesdits moyens se présentant sous forme d'orifices latéraux (7) percés à travers ledit canal d'évacuation.

2. Dispositif selon la revendication 1, **caractérisé en ce que** ledit orifice micrométrique est formé par l'extrémité proximale (6p) d'un conduit micrométrique (6).

3. Dispositif selon la revendication 1, **caractérisé en ce que** ledit orifice micrométrique (6p) est formé dans un obturateur (5) rapporté dans ledit canal de soufflage (3).

4. Dispositif selon la revendication 2, **caractérisé en ce que** ledit conduit micrométrique (6) est formé dans un obturateur (5) rapporté dans ledit canal de soufflage (3).

5. Dispositif selon l'une des revendications 1 à 4, **caractérisé en ce qu'**il comporte une pièce tubulaire (2, 3, 4) en forme de Y, dont les branches constituent respectivement le canal d'aspiration (2) et le canal de soufflage (3) et dont le tronc commun forme ledit canal d'évacuation (4).

6. Dispositif selon l'une des revendications 1 à 5, **caractérisé en ce qu'**il est associé à un récipient de collecte (10) dans lequel pénètre l'extrémité proximale (4p) dudit canal d'évacuation (4) et qui comporte au moins un évent (12).

7. Dispositif selon la revendication 6, **caractérisé en ce que** ledit récipient de collecte (10) présente la forme d'une poche et **en ce que** ledit dispositif d'aspiration (1) est solidaire de ladite poche (10).

## Claims

1. A suction devise (1) for medical applications, including:
- a suction channel (2), whose distal end (2d) is adapted to be connected to a patient (P); and
- means for generating a vacuum in said suction channel (2) by means of a pressurized gas,
**characterized in that**:
- said means for generating said vacuum include a micrometric hole (6p), arranged in a blowing channel (3), whose axis (s-s) converges towards that (a-a) of said suction channel (2), said micrometric hole (6p) being supplied with pressurized gas from the side opposite to said suction channel (2);
- the blowing channel converges towards the proximal end (2p) of said suction channel (2), the distal end (3d) of said suction channel (3) being adapted to be connected to a pressurized gas source (G);
- an exhaust channel (4) is connected to the proximal ends (2p, 3p) of said suction channel (2) and said blowing channel (3) to extend these two channels (2,3) on the proximal side; and
- the proximal end (4p) of said exhaust channel (4) is provided with means (7) for preventing the accidental plugging thereof, said means being in the form of side holes (7) pierced through said exhaust channel.

2. The device according to claim 1, **characterized in that** said micrometric hole is formed by the proximal end (6p) of a micrometric duct (6).

3. The device according to claim 1, **characterized in that** said micrometric hole (6p) is formed in a shutter (5) added in said blowing channel (3).

4. The device according to claim 2, **characterized in that** said micrometric duct (6) is formed in a shutter (5) added in said blowing channel (3).

5. The device according to one of claims 1 to 4, **characterized in that** it includes a Y-shaped tubular part (2, 3, 4), whose legs form the suction channel (2) and the blowing channel (3), respectively, and whose common trunk forms said exhaust channel (4).

6. The device according to one of claims 1 to 5, **characterized in that** it is associated with a collecting vessel (10) into which enters the proximal end (4p) of said exhaust channel (4), and which comprises at least one vent (12).

7. The device according to claim 6, **characterized in that** said collecting vessel (10) is in the form of a bag and **in that** said suction device (1) is integral with said bad (10).

## Patentansprüche

1. Saugvorrichtung (1) für medizinische Anwendungen, welche aufweist:
- einen Saugkanal (2), dessen distales Ende (2d) geeignet ist, mit einem Patienten (P) verbunden zu werden; und
- Mittel zum Erzeugen eines Unterdruckes in dem Saugkanal (2) mit Hilfe eines Druckgases,
**dadurch gekennzeichnet, dass**:
- die Mittel zum Erzeugen des Unterdruckes eine mikrometrische Öffnung (6p) aufweisen, die in einem Blaskanal (3) angeordnet ist, dessen Achse (s-s) sich mit derjenigen (a-a) des Saugkanals (2) schneidet, wobei die mikrometrische Öffnung (6p) von der dem Saugkanal (2) gegenüberliegenden Seite her mit Druckgas gespeist wird,
- der Blaskanal in Richtung des proximalen Endes (2p) des Saugkanals (2) verläuft, wobei das distale Ende (3d) des Blaskanals (3) geeignet ist, mit einer Druckgasquelle (G) verbunden zu werden;
- ein Auslasskanal (4) mit den proximalen Enden (2p, 3p) des Saugkanals (2) und des Blaskanals (3) verbunden ist, um diese beiden Kanäle (2, 3) auf der proximalen Seite zu verlängern; und
- das proximale Ende (4p) des Auslasskanals (4) mit Mitteln (7) versehen ist, die seine zufällige Verstopfung verhindert, wobei diese Mittel die Form von seitlichen Öffnungen (7) ausweisen, die durch den Auslasskanal hindurchführen.

2. Vorrichtung nach Anspruch 1, **dadurch gekennzeichnet, dass** die mikrometrische Öffnung von dem proximalen Ende (6p) einer mikrometrischen Leitung (6) gebildet wird.

3. Vorrichtung nach Anspruch 1, **dadurch gekennzeichnet, dass** die mikrometrische Öffnung (6p) in einem Verschlussstück (5) ausgebildet ist, das in den Blaskanal (3) eingesetzt ist.

4. Vorrichtung nach Anspruch 2, **dadurch gekennzeichnet, dass** die mikrometrische Leitung (6) in einem Verschlussstück (5) ausgebildet ist, das in den Blaskanal (3) eingesetzt ist.

5. Vorrichtung nach einem der Ansprüche 1 bis 4, **dadurch gekennzeichnet dass** sie ein rohrförmiges Teil (2, 3, 4) von der Form eines "Y" aufweist, dessen Schenkel den Saugkanal (2) bzw. den Blaskanal (3) bilden und dessen gemeinsamer Schaft den Auslasskanal (4) bildet.

6. Vorrichtung nach einem der Ansprüche 1 bis 5 **dadurch gekennzeichnet, dass** sie einem sammelbehälter (10) zugeordnet ist, in welchen das proximale Ende (4p) des Auslasskanals (4) hineinragt und welcher mindestens eine Entlüftungsöffnung (12) ausweist.

7. Vorrichtung nach Anspruch 6, **dadurch gekennzeichnet, dass** der Sammelbehälter (10) die Form eines Beutels aufweist, und dadurch, dass die Saugvorrichtung (1) mit dem Beutel (10) fest verbunden ist.
